# FASCICULE DE BREVET EUROPEEN

(11) **EP 3 519 381 B1**
(45) Date de publication et mention de la délivrance du brevet: **26.08.2020**
(21) Numéro de dépôt: 17783947.9
(22) Date de dépôt: 29.09.2017
(51) Int. Cl.: C07C 29/76, C07C 31/30

(54) **PROCÉDÉ DE FABRICATION DE DIGLYCEROXYDE DE CALCIUM**
VERFAHREN ZUR HERSTELLUNG VON CALCIUMDIGLYCEROXID
METHOD FOR MANUFACTURING CALCIUM DIGLYCEROXIDE

(30) Priorité: 29.09.2016 FR 1659382
(43) Date de publication de la demande: 07.08.2019
(73) Titulaire: Easyl, 74130 Bonneville (FR)
(72) Inventeur: LACOSTE, François, 92200 Neuilly Sur Seine (FR); LAIR, Valentin, 52000 Chaumont (FR); THIEL, Julien, 74930 Arbusigny (FR); HALLOUMI, Samy, 74800 La Roche Sur Foron (FR)
(74) Mandataire: Jacobacci Coralis Harle
(86) Numéro de dépôt international: PCT/FR2017/052675
(87) Numéro de publication internationale: WO 2018/060654

(56) Documents cités:
- LUKIC, IVANA ET AL: "Calcium diglyceroxide synthesized by mechanochemical treatment, its characterization and application as catalyst for fatty acid methyl esters production", FUEL, vol. 165, 23 octobre 2015 (2015-10-23), pages 159-165, XP002770448, ISSN: 0016-2361
- RASHMI SHARMA ET AL.: "Nanoparticule technology: formulating poorly water-soluble compounds: a review", INTERNATIONAL JOURNAL OF PHARMACEUTICAL SCIENCES AND RESEARCH, vol. 6, no. 1, 1 janvier 2015 (2015-01-01) , pages 57-71, XP002770498, ISSN: 2320-5148

## Description

### DOMAINE TECHNIQUE AUQUEL SE RAPPORTE L'INVENTION

La présente invention se rapporte à un procédé de fabrication de cristaux de diglycéroxyde de calcium.

En particulier, la présente invention concerne un procédé de fabrication de cristaux de diglycéroxyde de calcium, effectué par microbroyage, notamment sans chauffage particulier, d'un mélange d'au moins un composé source de calcium et de glycérol, et éventuellement d'un solvant anhydre du glycérol.

Les cristaux de diglycéroxyde de calcium obtenus avec le procédé susmentionné peuvent être utilisés comme catalyseur hétérogène pour la production de biodiesel, et plus particulièrement pour la réaction de transestérification des triglycérides.

### ARRIERE-PLAN TECHNOLOGIQUE

Le diglycéroxyde de calcium, de formule Ca(C₃H₇O₃)₂ a fait l'objet de recherches, notamment en vue de son utilisation en tant que catalyseur de la réaction de transestérification des triglycérides.

Lukic et al. dans l'article « Calcium diglyceroxide synthesized by mechanochemical treatment, its characterization and application as catalyst for fatty acid, methyl esters production », Fuel 165(2016), 159-165 présente dans le tableau 1, page 160, les procédés connus de synthèse du diglycéroxyde de calcium. Cet article décrit également la préparation de diglycéroxyde de calcium, nommée, à partir de CaO et de glycérol selon un ratio molaire de 1 :5. Le CaO et le glycérol subissent un traitement mécano-chimique dans un broyeur planétaire à billes Fritsch (Pulvérisette 5) (2 flacons de 500 cm³ comprenant 450 g de billes de 10 mm de diamètre). Le broyage dure 5 heures.

Ainsi, ces procédés souffrent du fait qu'ils nécessitent soit une température élevée (supérieure à 50°C), soit d'un long temps de réaction (5 heures) ou les deux à la fois.

La publication de Changjun Li et al. « Transesterification of jatropha oil to biodiesel by using catalyst containing Ca(C2H7O3)2 as a solid base catalyst », Advanced materials research, ISSN: 1662-8985, vol.666, pp 93-102, décrit la préparation de diglycéroxyde de calcium à partir d'une part, de KOH et de glycérol qui sont chauffés à 140°C sous agitation constante jusqu'à dissolution de KOH et d'autre part, de CaCl₂ qui est dissous dans du méthanol anhydre. Les deux solutions sont ensuite mélangées (température inférieure à 40°C) afin de réaliser une réaction par précipitation.

Par conséquent, cette publication décrit la fabrication de diglycéroxyde de calcium par voie chimique nécessitant plusieurs étapes (préparation de deux solutions) et une température élevée, ici 140°C.

On connaît également de l'état de la technique des publications plus générales portant sur les nanoparticules.

A titre d'exemple, la publication de Sharma et al. « Nanoparticle technology : formulating poorly water-soluble compounds : a review », International journal of pharmaceutical sciences and research, 2015; Vol.6(1):57-71, répertorie différentes méthodes afin d'obtenir des nanoparticules. En effet, il est indiqué que l'efficacité thérapeutique d'un médicament dépend de sa biodisponibilité et de sa solubilité dans l'eau et que ces propriétés peuvent être améliorées au stade de nanoparticules. Dans les différentes méthodes décrites dans ce document afin de réduire la taille d'un principe actif de départ (pas de réaction chimique), il est mentionné le broyeur à microbilles en phase humide Dyno Mil.

On connaît également de l'état de la technique le document EP 2 323 964 qui décrit un procédé afin de produire des glycéroxydes de métal à partir de glycérol humide en tant que sous-produit de la production de bio-diesel et d'un hydroxyde de métal, comme l'hydroxyde de calcium. Le procédé a lieu sous agitation via par exemple des lames rotatives (mixeur alimentaire Kenwood kMix). Ce procédé produit une réaction exothermique (température allant de 90 à 140°C) dans laquelle l'hydroxyde de calcium et l'eau présente dans le glycérol vont réagir.

Par conséquent, ce procédé présente l'inconvénient de se réaliser à des températures élevées.

La présente invention a donc pour objet de fournir un procédé de fabrication de diglycéroxyde de calcium simple, industriellement exploitable, notamment ne nécessitant pas de chauffage important avec un temps de réaction extrêmement court.

### OBJET DE L'INVENTION

A cet effet, la présente invention fournit un procédé de fabrication de cristaux de diglycéroxyde de calcium qui comprend au moins les étapes suivantes :
(1) la mise en suspension d'au moins un composé source de l'élément calcium, notamment l'oxyde de calcium (CaO), dans du glycérol ou dans un mélange homogène de glycérol (C₃H₈O₃) et d'un solvant anhydre du glycérol, notamment du méthanol (CH₃OH), dite « suspension de départ », le ratio molaire (glycérol) : (composé(s) source de l'élément calcium) étant supérieur ou égal à 2, de préférence de 2 à 10, et en particulier de 2 à 5 ;
(2) le broyage de ladite suspension de départ à une température ambiante inférieure ou égale à 50°C, de préférence inférieure ou égale à 35°C, dans un broyeur tridimensionnel à microbilles en phase liquide pendant un temps de séjour inférieur ou égal à 15 min, de préférence inférieur ou égal à 1 minute, et allant notamment de 5 à 25 secondes et en particulier de 10 à 20 secondes ;
(3) la récupération en sortie du broyeur d'une suspension de cristaux de diglycéroxyde de calcium ; et
(4) optionnellement, un lavage de la suspension obtenue avec un solvant du glycérol afin d'éliminer le possible excès de glycérol ;
(5) optionnellement, ladite suspension de cristaux de diglycéroxyde de calcium est séchée de sorte à obtenir une poudre de cristaux de diglycéroxyde de calcium.

Le ratio molaire solvant anhydre du glycérol (notamment le méthanol) : glycérol peut varier de 0 à 30, plus particulièrement de 2 à 15 et de préférence de 3 à 12.

Parmi les solvants anhydres du glycérol, on peut citer les alcanols, notamment le méthanol, l'isopropanol et l'éthanol. Le solvant préféré est le méthanol.

En particulier, la suspension de départ ne comprend pas d'eau ou seulement à l'état de traces, à savoir en une proportion inférieure ou égale à 3 %, de préférence inférieure ou égale à 1 % et typiquement inférieure ou égale à 0,3%, en masse, par rapport à la masse totale de la suspension de départ.

Selon l'invention, « une proportion inférieure ou égale à 3 % » comprend les valeurs suivantes : 3 ; 2 ; 1 ; 0,5 ; 0,4 ; 0,3 ; 0,2 ; 0,1 ; 0 ou tous intervalles situés entre ces valeurs.

Dans la présente invention, on entend par composé « source de l'élément calcium », tout composé contenant l'élément calcium et apte à fournir lors de la synthèse du diglycéroxyde la quantité d'élément calcium nécessaire à la formation du calcium diglycéroxyde.

Parmi les composés source de l'élément calcium, on peut citer CaO ou Ca(OH)₂, l'acétate de calcium ou encore le méthanolate de calcium, etc. ou une de leurs combinaisons

Le composé source de calcium préféré est CaO.

La présente invention permet ainsi à partir d'au moins deux composants de départ qui sont distincts, généralement non nanométriques et non thermosensibles, d'obtenir du diglycéroxyde de calcium en une seule étape.

La présente invention a également pour objet des cristaux de diglycéroxyde de calcium obtenus selon le procédé décrit ci-dessus.

Les cristaux de diglycéroxyde de calcium obtenus selon le procédé décrit ci-dessus peuvent être notamment utilisés pour fabriquer un catalyseur hétérogène pour la production de biodiesel.

Pour le reste de la description, à moins qu'il n'en soit spécifié autrement, l'indication d'un intervalle de valeurs « de X à Y » ou « entre X et Y », dans la présente invention, s'entend comme incluant les valeurs X et Y.

### DESCRIPTION DETAILLEE D'UN EXEMPLE DE REALISATION

L'invention sera mieux comprise et d'autres buts, détails, caractéristiques et avantages de celle-ci apparaîtront plus clairement à la lecture de la description suivante d'exemples de réalisation, en référence aux figures annexées dans lesquelles :
- la figure 1 représente une vue en coupe selon l'axe longitudinal XX d'un broyeur tridimensionnel à microbilles en phase liquide, selon une variante de réalisation convenant pour la mise en œuvre du procédé selon l'invention ;
- la figure 2 représente des vues en coupe selon l'axe XX et l'axe AA, de variantes de broyeurs tridimensionnels à microbilles en phase liquide selon la figure 1 dans lesquels : (a) l'agitateur est à disques, (b) l'agitateur comporte des doigts et (c) la chambre de broyage est annulaire ;

- la figure 3 est une photo de microscopie à balayage électronique (MEB) de cristaux de diglycéroxyde de calcium obtenus selon le procédé de l'invention en utilisant les paramètres suivants : suspension de départ comprenant 980g de matières premières (glycérol + CaO) avec un ratio molaire (glycérol) : (CaO) égal à 5, dans 1kg de solvant anhydre du glycérol (ici, le méthanol) ; un débit de passage de la suspension de départ dans le broyeur de 45L/h et un diamètre de billes de 500 µm ;
- la figure 4 est un spectre de diffractométrie à rayons X (DRX) de cristaux de diglycéroxyde de calcium obtenus dans les mêmes conditions expérimentales que ceux de la figure 3 ;
- la figure 5 sont des courbes ATD/ATG des diglycéroxyde de calcium obtenu ; et
- les figures 6, 7 et 8 sont des microphotographies MEB des cristaux de diglycéroxyde de calcium obtenus en utilisant respectivement des billes de zircone de 0,5 mm et 2 mm et des billes de silice de 2 mm.
- la figure 9 est un spectre de diffractométrie à rayons X (DRX) de cristaux de diglycéroxyde de calcium obtenus selon le procédé de l'invention en utilisant les paramètres suivants: suspension de départ comprenant 1072g de matières premières (glycérol + Ca(OH)₂) avec un ratio molaire (glycérol) : (Ca(OH)₂) égal à 5, dans 1kg de solvant anhydre du glycérol (ici, le méthanol) ; un débit de passage de la suspension de départ dans le broyeur de 45L/h et un diamètre de billes de 500 µm ;

La Demanderesse s'est attachée au développement d'un nouveau procédé de fabrication de cristaux de diglycéroxyde de calcium adapté pour être mis en œuvre à une échelle industrielle et permettant notamment, à titre d'exemple, dans le cas de l'emploi de CaO comme source de calcium, la synthèse suivante :

CaO + 2 C₃H₈O₃ → Ca(C₃H₇O₃)₂ + H₂O.

En particulier, la présente invention a pour objet un procédé de fabrication de cristaux de diglycéroxyde de calcium qui comprend au moins les étapes suivantes :
(1) la mise en suspension d'au moins un composé minéral contenant l'élément calcium (CaO, Ca(OH)₂...), du glycérol ou dans un mélange homogène de glycérol (C₃H₈O₃) et d'un solvant anhydre du glycérol, par exemple du méthanol, dite « suspension de départ », le ratio molaire (solvant anhydre du glycérol): (glycérol) allant de 0 à 30, plus particulièrement de 2 à 15, et de préférence de 3 à 12. Le ratio molaire (glycérol) : (composé(s) source de l'élément calcium) étant supérieur ou égal à 2, de préférence de 2 à 10 et en particulier de 2 à 5 ;
(2) le broyage de ladite suspension de départ à une température ambiante inférieure ou égale à 50°C, de préférence inférieure ou égale à 35°C, dans un broyeur tridimensionnel à microbilles en phase liquide pendant un temps de séjour inférieur ou égal à 15 min, de préférence inférieur ou égal à 1 minute, et allant notamment de 5 à 25 secondes et en particulier de 10 à 20 secondes ;
(3) la récupération en sortie du broyeur d'une suspension de cristaux de diglycéroxyde de calcium ; et
(4) optionnellement, un lavage de la suspension obtenue avec un solvant du glycérol (par exemple méthanol) afin d'éliminer le possible excès de glycérol.
(5) optionnellement, ladite suspension de cristaux de diglycéroxyde de calcium est séchée de sorte à obtenir une poudre de cristaux de diglycéroxyde de calcium.

Selon l'invention, « une température ambiante inférieure ou égale à 50°C», comprend les valeurs suivantes : 50 ; 49 ; 48 ; 47 ; 46 ; 45 ; 44 ; 43 ; 42 ; 41 ; 40 ; 39 ; 38 ; 37 ; 36 ; 35 ; 34 ; 33 ; 32 ; 31 ; 30 ; 29 ; 28 ; 27 ; 26 ; 25 ; 24 ; 23 ; 22 ; 21 ; 20 ; 19 ; 18 ; 17 ; 16 ; 15 ; 14 ; 13 ; 12 ; 11 ; 10 ; etc. ou tous intervalles situés entre ces valeurs.

Egalement, selon l'invention, « un temps de séjour inférieur ou égal à 15 min » comprend les valeurs suivantes : 15 min; 14 min; 13 min ; 12 min ; 11 min; 10 min; 9 min; 8 min; 7 min ; 6 min; 5 min; 4 min; 3 min; 2 min ; 1 min ; 55 sec ; 50 sec ; 45 sec ; 40 sec ; 35 sec ; 30 sec ; 25 sec ; 20 sec ; 15 sec ; 10 sec ; 5 sec ; etc. ou tous intervalles situés entre ces valeurs.

La Demanderesse a ainsi mis au point un procédé qui, de façon inattendue, permet de fabriquer des cristaux de diglycéroxyde de calcium en un temps très court (temps de réaction inférieur ou égal à 15 minutes et en général, inférieur ou égal à 1 minute), en une seule étape, à température ambiante (le procédé ne nécessite pas d'étape de chauffage particulière), et ce avec une faible consommation d'énergie et en outre un excellent rendement.

Comme cela sera démontré dans les essais ci-dessous, le procédé de l'invention permet de plus d'obtenir de façon surprenante des cristaux de diglycéroxyde de calcium d'excellente qualité, à savoir très purs.

Le procédé selon l'invention présente également les avantages d'avoir un prix de revient très réduit (les matières premières utilisées sont en effet largement disponibles, non polluantes et peu coûteuses) et de présenter une excellente reproductibilité, ce qui le démarque davantage des procédés décrits dans les arts antérieurs. Le procédé selon l'invention présente également l'avantage de pouvoir être mis en œuvre de façon continue. Or, ces caractéristiques sont importantes pour une application à l'échelle industrielle.

En outre, malgré les nombreuses recherches conduites sur la synthèse de cristaux de diglycéroxyde de calcium, aucune n'a suggéré le procédé susmentionné et en particulier une étape de broyage dans un broyeur tridimensionnel à microbilles à partir d'une suspension de départ comprenant de l'oxyde de calcium (CaO) ou une autre source de calcium et du glycérol (C₃H₈O₃), et ce, en présence ou non d'un solvant anhydre du glycérol, tel que du méthanol (CH₃OH).

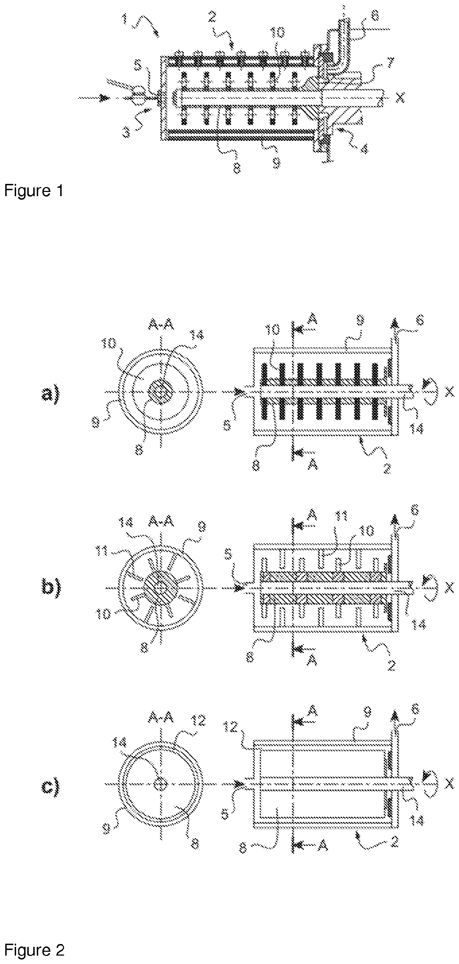

Pour mieux faire comprendre le procédé objet de l'invention, un broyeur tridimensionnel à microbilles susceptible de permettre l'obtention des cristaux de diglycéroxyde de calcium, et faisant ainsi partie de l'invention, va tout d'abord être décrit ci-dessous en se référant aux figures 1 et 2.

Tel qu'illustré sur la figure 1, un broyeur tridimensionnel à microbilles 1 comprend au moins :
- une chambre de broyage 2 stationnaire de forme générale cylindrique s'étendant selon un axe longitudinal XX, ladite chambre 2 étant remplie au moins, en partie, par lesdites microbilles (non représentées) et comprend: à une première extrémité 3 au moins une entrée 5 servant à introduire ladite suspension de départ, et à une seconde extrémité 4, une sortie 6 comportant un moyen de séparation 7 apte à n'évacuer que la suspension de diglycéroxyde de calcium ainsi formée dans ladite chambre 2; et
- un agitateur 8, disposé dans la chambre de broyage 2 stationnaire, se présentant sous forme d'une tige allongée selon l'axe longitudinal XX, ledit agitateur 8 étant apte à mettre en mouvement l'ensemble microbilles/suspension de départ.

En particulier, l'entrée 5 est généralement reliée à une pompe péristaltique (non représentée). Cette pompe permet d'amener la suspension de départ, contenue par exemple dans un récipient, tel qu'une cuve, à l'intérieur de la chambre de broyage 2 via l'entrée 5. La pompe permet en plus, lors du fonctionnement du broyeur tridimensionnel, d'amener cette suspension de départ selon un certain débit qui est réglable, appelé par la suite « débit de passage ». Ce débit de passage forme en outre un courant dans la chambre de broyage 2 permettant d'entraîner la suspension de départ de l'entrée 5 vers la sortie 6.

La sortie 6 de la chambre de broyage 2 comprend en particulier le système de séparation 7 des microbilles de la suspension finale comprenant majoritairement les cristaux de diglycéroxyde de calcium, du méthanol et éventuellement le solde de matières premières qui n'auraient pas réagi. Ce moyen de séparation 7 peut être un tamis dont les orifices présentent une dimension inférieure à celle des microbilles ou une fente de séparation dont la largeur est également adaptée pour retenir les microbilles au sein de la chambre 2.

La paroi interne 9 de la chambre de broyage 2 comprend selon un premier mode de réalisation une surface interne lisse. Cependant, selon une variante de réalisation qui sera décrite ci-après, il peut être aménagé sur cette surface interne 9 des doigts 11.

Tel que mentionné ci-dessus, à l'intérieur de la chambre de broyage 2 est disposé l'agitateur 8 qui, en plus du débit de passage, permet également la mise en mouvement de la suspension de départ.

En particulier, l'agitateur 8 est apte à tourner autour de l'axe X via un arbre rotatif (14, figure 2) pour impartir au sein de la chambre de broyage 2 un mouvement tourbillonnaire à la suspension de départ et effectuer ainsi un brassage intense entre cette suspension de départ et les microbilles présentes dans la chambre 2 le long de la paroi interne 9 de cette chambre 2.

En particulier, le broyeur via son arbre rotatif 14 présente une vitesse de rotation supérieure ou égale à 100 tours par minute, avantageusement de 1000 tours par minute, de préférence supérieure ou égale à 2000 tours par minute et typiquement supérieure ou égale à 2500 tours par minute.

Au sens de l'invention, « une vitesse de rotation supérieure ou égale à 100 tours par minutes » comprend les valeurs suivantes : 100 ; 150 ; 200 ; 250 ; 300 ; 350 ; 400 ; 450 ;500 ; 550 ;600 ; 650 ;700 ; 750 ;800 ; 850 ; 900, 950 ; 1000 ; 1100 ; 1200 ; 1300 ; 1400 ; 1500 ; 1600 ; 1700 ; 1800 ; 1900 ; 2000 ; 2100 ; 2200 ; 2300 ; 2400 ; 2500 ; 2600 ; 2700 ; 2800 ; 2900 ; 3000 ; 3100 ; 3200 ; 3300 ; 3400 ; 3500 ; 3600 ; 3700 ; 3800 ; 3900 ; 4000, 4500 ; 5000 ; 5500 ; 6000 ; etc., ou tous intervalles compris entre ces valeurs.

En général, le broyeur présente une vitesse de rotation allant de 1000 tr/min à 5000 tr/min, en particulier de 1500 tr/min à 4500 tr/min, de préférence de 2000 tr/min à 4000 tr/min et typiquement de 2800 à 3200 tr/min.

Afin d'améliorer ce brassage, l'agitateur 8, tout comme la paroi interne 9 de la chambre 2, peuvent présenter diverses configurations possibles représentées par exemple sur la figure 2.

Selon une première configuration illustrée à la figure 2a, l'agitateur 8 comprend le long de sa tige allongée des disques 10, disposés perpendiculairement à celle-ci. Leur nombre peut varier de 2 à 8, de préférence de 2 à 5. Ces disques 10 permettent d'une part, d'améliorer le broyage de la suspension de départ en brassant davantage les microbilles et d'autre part, d'accélérer le temps de réaction.

Selon une deuxième configuration illustrée à la figure 2b, l'agitateur 8 peut également comprendre le long de sa tige un ou plusieurs disques 10 disposés perpendiculaires et qui sont en outre aptes à coopérer avec des doigts 11, disposés perpendiculairement, par rapport à la paroi interne 9 de la chambre 2. Un doigt se présente notamment sous la forme d'un anneau qui s'étend perpendiculairement à partir de la paroi 9. Pour cette configuration, les disques 10 et les doigts 11 sont disposés en quinconce, à savoir les disques 10 et les doigts 11 sont disposés de manière alternée dans la chambre 2. En outre, l'épaisseur de la tige 8 est augmentée par rapport à la configuration précédente (figure 2a) de sorte que la périphérie des disques 10 soit proche de la paroi interne 9 et que celle des doigts 11 soit proche de la périphérie de la tige de l'agitateur 8. Ainsi, dans cette configuration, le volume de la chambre est réduit par rapport à la configuration précédente, permettant par conséquent, un meilleur brassage entre la suspension de départ, les microbilles et la paroi interne 9 de la chambre 2.

Le volume de la chambre 2 peut encore être réduit comme cela est illustré en figure 2c. Dans cette configuration, l'agitateur 8 présente un diamètre externe légèrement inférieur au diamètre interne de la chambre 2, formant ainsi une chambre annulaire 12 de faible volume disposée entre la paroi externe de l'agitateur 8 et la paroi interne 9 de la chambre 2. Les microbilles (non représentées) sont disposées dans cette chambre annulaire 12. Lors du fonctionnement de cette configuration, la suspension de départ est introduite par l'entrée 5 avec un certain débit, qui va ensuite parcourir la chambre annulaire 12 jusqu'à la sortie 6 tout en étant brassée par les microbilles.

En général, le broyeur convenant pour réaliser le procédé selon l'invention comprend une chambre de broyage présentant un diamètre de 75 mm à 300 mm pour une longueur de 80 mm à 900 mm et un agitateur présentant une taille allant de 65 mm à 260 mm. Ainsi, le volume de la chambre de broyage varie de 0,35 L à 600 L, de préférence de 0,35 L à 400 L, et typiquement de 0,35 L à 62 L.

Au sens de l'invention, « un volume de la chambre de broyage allant de 0,35 L à 600 L» comprend les valeurs suivantes : 0,35 ; 0,5 ; 0,8 ; 1 ; 2 ; 3 ; 4 ; 5 ; 6 ; 7 ; 8 ; 9 ; 10 ; 15 ; 20 ; 25 ; 30 ; 35 ; 40 ; 45 ; 50 ; 55 ; 60 ; 65 ; 70 ; 80 ; 85 ; 90 ;100 ; 110 ; 120 ; 130 ; 140 ; 150 ; 160 ; 170 ; 180 ; 190 ; 200 ; 210 ; 220 ; 230 ; 240 ; 250 ; 260 ; 270 ; 280 ; 290 ; 300 ; 350 ; 400 ; 450 ; 500 ; 550 ; 600 etc., ou tous intervalles compris entre ces valeurs.

La géométrie de la chambre de broyage et de l'agitateur pourra être ajustée par l'homme du métier en fonction de la quantité de cristaux de diglycéroxyde de calcium souhaitée, ainsi que du temps de réaction désiré. Par exemple, il est également possible que la chambre de broyage 2 comprenne un accélérateur afin d'améliorer le broyage de la suspension de départ.

En outre, les microbilles logées dans la chambre de broyage 2 et convenant pour le procédé selon l'invention sont substantiellement de forme sphérique et présentent un diamètre moyen inférieur ou égal à 5 mm, généralement allant de 0,05 mm à 4 mm, de préférence de 0,2 à 3 mm, en particulier de 0,3 à 2 mm, et typiquement de l'ordre de 0,5 à 1 mm. De préférence, le diamètre des microbilles est inférieur ou égal à 1 mm et est typiquement de l'ordre de 0,05 mm à 1 mm.

Elles sont préférentiellement choisies parmi des microbilles présentant une dureté élevée et résistant relativement bien à l'abrasion.

En particulier, les microbilles présentent une dureté de Vickers mesurée selon la norme EN ISO 6507-1 supérieure ou égale à 900 HV1, de préférence allant de 900 HV1 à 1600 HV1, typiquement allant de 1000 à 1400 HV1 et notamment allant de 110 à 1300 HV1.

Au sens de l'invention, « une dureté de Vickers supérieure ou égale à 900 HV1» comprend les valeurs suivantes : 900 ; 910 ; 920 ; 930 ; 940 ; 950 ; 960; 970; 980; 990 ; 1000; 1010; 1020; 1030; 1040; 1050; 1060; 1070; 1080 ; 1090 ; 1000 ; 1110 ; 1120 ; 1130 ; 1140 ; 1150 ; 1160 ; 1170 ; 1180 ; 1190 ; 1200 ; 1300 ; 1400 ; 1500 ; 1600 ; 1700 ; etc., ou tous intervalles compris entre ces valeurs.

Avantageusement, elles présentent une masse volumique réelle élevée. En général, les microbilles selon l'invention ont une masse volumique réelle supérieure ou égale 2 g/cm³, en particulier allant de 2 à 15 g/cm³, de préférence de 3 à 12 g/cm³, et typiquement de 4 à 10 g/cm³.

Ainsi, les microbilles selon l'invention peuvent être des microbilles en céramique, (oxyde de zirconium ZrO₂, en silicate de zirconium ZrSiO₄) ; des microbilles en acier, des microbilles en carbure de tungstène, des microbilles en verre ou une de leurs combinaisons.

De préférence, les microbilles sont en céramiques car elles ne génèrent pas de pollution par leur usure.

En particulier, les microbilles sont en oxyde de zirconium.

Eventuellement, les microbilles en oxyde de zirconium peuvent être stabilisées par un autre oxyde, tel que l'oxyde de cérium, l'oxyde d'yttrium et/ou le silicium.

A titre d'exemples, les compositions suivantes, résumées dans le tableau 1 ci-dessous, conviennent pour former les microbilles selon l'invention :

**Tableau 1**

| **Composition des microbilles** | **Dureté HV1** | **Masse volumique réelle (g/cm³)** | **Fabricant** |
|---|---|---|---|
| Microbilles en oxyde de zirconium stabilisées par l'oxyde de cérium | 1180 | ≥6,10 | Saint-Gobain (Zirmil®Y Ceramic Beads) ou EIP (Procerox® ZO Cer) |
| - 80% de ZrO₂ | | | |
| - 20% de CeO | | | |
| Microbilles en oxyde de zirconium stabilisées par l'yttrium | 1250 | ≥ 5,95 | EIP (Procerox® ZO (Y)) |
| - 95% ZrO₂ | | | |
| - <5% Al₂O₃ | | | |
| - Reste : Y₂O₃ | | | |
| Microbilles en oxyde de zirconium stabilisées par l'yttrium et du silicium : | > 700 | >4,80 | Saint-Gobain (ER120 Ceramic Beads) |
| - 78% ZrO₂, | | | |
| - 12% SiO₂, | | | |
| - 5% Al₂O₃ et | | | |
| - 4% Y₂O₃ | | | |
| Microbilles en silicate de zirconium ZrSiO₄ | ≥ 800 | > 6,5 | Saint-Gobain (Rimax Ceramic Beads) |
| Microbilles en verre | 500 | > 3,76 | - |
| Microbilles en acier | 700 | > 7,7 | - |

Généralement, les microbilles convenant pour le procédé de l'invention ne sont pas en verre ou exclusivement en verre.

En particulier, les microbilles représentent, en volume, par rapport au volume total de la chambre stationnaire 2 de 50% à 85%, de préférence de 55% à 70%.

Au sens de l'invention, « un volume de 50 à 85 % » comprend les valeurs suivantes : 50 ; 55 ; 60 ; 65 ; 70 ; 75 ; 80 ; 85; etc., ou tous intervalles compris entre ces valeurs.

A titre d'exemple, le broyeur tridimensionnel à microbilles en phase liquide convenant pour réaliser le procédé selon l'invention peut correspondre à des broyeurs commercialisés par les sociétés WAB, gamme Dyno-Mill : Multi Lab, ECM et KD, NETZCH ou encore Alpine Hosokawa, par exemple, Agitated Media Mill AHM.

Le procédé de fabrication selon l'invention va désormais être décrit plus explicitement ci-dessous.

Tel qu'indiqué précédemment, la fabrication des cristaux de diglycéroxyde de calcium selon l'invention comprend tout d'abord (1) une mise en suspension d'un composé source d'élément Ca, par exemple de l''Oxyde de calcium (CaO), ou un composé minéral contenant l'élément calcium (Ca(OH)₂...) dans du glycérol (C₃H₈O₃), éventuellement en mélange homogène avec un solvant anhydre du glycérol tel que du méthanol (CH₃OH), dite « suspension de départ ».

La suspension de départ est classiquement préparée par mélange des matières premières (CaO + C₃H₈O₃) avec ou non le solvant anhydre (méthanol) dans un dispositif approprié, tel qu'un récipient ou une cuve, muni d'un système d'agitation (tel qu'agitateur magnétique, des pales d'agitation, etc.). Le dispositif ainsi que le système d'agitation pourront être adaptés par l'homme du métier en fonction de la quantité de cristaux de diglycéroxyde de calcium à fabriquer.

De préférence, le composé source de l'élément calcium et le glycérol sont mélangés en proportion stœchiométrique.

En particulier, l'oxyde de calcium et le glycérol sont mélangés, de préférence, en proportion stœchiométrique dans la suspension de départ pour réaliser la réaction :

CaO + 2 C₃H₈O₃ → Ca(C₃H₇O₃)₂ + H₂O

Ceci correspond, en proportions massiques à 56 kg de CaO pour 184 kg de glycérol, soit 23.3% de CaO et 76.6% de glycérol. Ainsi, la suspension de départ est généralement exempte d'eau ou celle-ci n'est présente qu'à l'état de traces au sein de la suspension.

Il est bien sûr possible de s'écarter sensiblement de la proportion stœchiométrique, par exemple, en mélangeant de 18 à 28%, en masse de CaO, avec respectivement de 72 à 82% en masse de glycérol, par rapport au poids total de CaO + glycérol, si l'on désire par exemple une composition de diglycéroxyde de calcium contenant un excédent soit de glycérol, soit d'oxyde de calcium.

La présence du méthanol, non nécessaire à la réaction (optionnel), favorise la mise en mouvement des microbilles du broyeur pour un meilleur broyage de la suspension de départ et donc une meilleure synthèse des cristaux de diglycéroxyde de calcium.

En général, le composé source de l'élément calcium de la suspension de départ se trouve sous forme de poudre. Cette poudre a une taille de particules inférieure ou égale à 100 µm, de préférence inférieure ou égale à 50 µm, en particulier inférieure ou égale à 30 µm, telle que 20 µm ou 10 µm. Ainsi, il n'est pas nécessaire que la source de l'élément calcium soit à l'échelle nanométrique.

L'oxyde de calcium convenant pour la présente invention présente de préférence une taille de particules inférieure ou égale à 200 µm et de préférence inférieure ou égale à 100 µm, et en particulier la taille des particules va de 0.01 µm à 20 µm. L'oxyde de calcium de numéro CAS : 1305-78-8 et commercialisé par exemple par la société Sigma Aldrich de pureté supérieure ou égale à 99% convient pour réaliser le procédé de l'invention.

Le glycérol convenant pour la présente invention se trouve sous forme de liquide. Le glycérol de numéro CAS : 56-81-5 et commercialisé par exemple par la société Mon-droguiste.com de pureté supérieure ou égale à 99,5% convient pour réaliser le procédé de l'invention.

De préférence, l'oxyde de calcium et le glycérol présentent une pureté élevée, en général supérieure ou égale à 90%, en particulier supérieure ou égale à 95% et typiquement supérieure ou égale à 99%, voire supérieure ou égale à 99,9%.

Une fois que la suspension de départ est préparée, celle-ci est amenée au broyeur tridimensionnel à microbilles 1 par l'intermédiaire généralement de la pompe péristaltique à débit réglable via l'entrée 5. La pompe péristaltique permet de continuer le mélange de la suspension de départ avant l'entrée dans la chambre 2. En outre, tel qu'indiqué précédemment, cette pompe permet d'introduire la suspension de départ dans la chambre 2 avec un débit de passage contrôlé.

Généralement, la suspension de départ est introduite à un débit de passage supérieur ou égal à 10 L/h.

Au sens de l'invention, « un débit de passage supérieur ou égal à 10 L/h» comprend les valeurs suivantes : 10 L/h; 15 L/h ; 20 L/h ; 25 L/h ; 30 L/h ; 35 L/h ; 40 L/h; 45 L/h; 55 L/h; 60 L/h; 65 L/h; 70 L/h; 80 L/h ; 85 L/h ; 90 L/h; 95 L/h; 100 L/h ; 110 L/h; 120 L/h ; 130 L/h ; 140 L/h ; 150 L/h ; 50 L/h ; 55 L/h ; 60 L/h ; 65 L/h; 70 L/h; 75 L/h ; 80 L/h; 85 L/h; 90 L/h; 95 L/h; 100 L/h; 105 L/h ; 110 L/h; 115 L/h; 120 L/h ; 125 L/h ; 130 L/h; 135 L/h ; 140 L/h ; 145 L/h ; 150 L/h ; 155 L/h; 160 L/h; 165 L/h ; 170 L/h ; 175L/h ; 180 L/h ; 200 L/h ; 300 L/h ; 400 L/h ; 500 L/h ; 600 L/h ; 700 L/h ; 800 L/h ; 900L/h ; 1 m³/h ; 2 m³/h ; 3 m³/h ; 4 m³/h ; 5 m³/h ; 6 m³/h ; 7 m³/h ; 8 m³/h ; 9 m³/h ; 10 m³/h ; 11 m³/h ; 12 m³/h ; 13 m³/h ;14 m³/h ;15 m³/h ; etc ; etc., ou tous intervalles compris entre ces valeurs.

En particulier, la suspension de départ est introduite à un débit de passage allant de 10 à 130 L/h, de préférence allant de 20 à 100 L/h et typiquement allant de 30 à 90 L/h.

Bien entendu, les débits de passage pourront varier en fonction de la dimension du broyeur tridimensionnel à microbilles utilisé pour réaliser le procédé. Par exemple, pour un broyeur tridimensionnel à microbilles présentant une chambre stationnaire 2 de 0,5L de volume, le débit de passage pourra être de l'ordre de 40 à 150L/h, tel qu'environ 45 L/h ; tandis que pour des broyeurs de plus grande dimension présentant en particulier un chambre stationnaire 2 de 60L, le débit pourra être de l'ordre de 2 à 15 m³/h, tel qu'environ 4 m³/h.

Une fois que la suspension de départ est introduite dans la chambre 2, l'étape de broyage (2) débute.

Sous l'effet du courant créé par le débit de passage, la suspension de départ parcourt la chambre stationnaire 2 de l'entrée 5 à la sortie 6, tout en étant mise en mouvement par l'agitateur 8 qui permet un brassage intense de cette suspension avec les microbilles et, le cas échéant, avec les disques 10, les doigts 11, etc., le long de la paroi interne 9 de la chambre 2.

La vitesse de rotation de l'agitateur pourra par exemple varier de 4 à 20 Pi rad/s, de préférence de 4 à 8 Pi rad/s.

Le temps de séjour de la suspension de départ est inférieur ou égal à 15 min, de préférence inférieur ou égal à 5 minutes, en particulier inférieur ou égal à 1 minute, et va notamment dans le broyeur de 5 à 25 secondes et en particulier de 5 à 20 secondes, tel que de 1 à 15 secondes. Il est en effet inhérent au volume apparent des billes et au débit de passage.

Par exemple, si le volume total apparent des billes est de 270 cm³ (billes de masse volumique apparente de 3,7 g/cm³) et que le débit d'introduction de la suspension est de 45 L/h, soit 12,45 cm³/s, alors le temps de séjour de la suspension dans la chambre 2 est estimé à environ 22 secondes. Par conséquent, le temps de séjour peut être avantageusement réglé, par exemple en contrôlant la masse volumique apparente des microbilles, ainsi que le débit de passage.

On entend par « volume apparent » le volume des microbilles incluant l'air interstitiel entre les billes. La masse volumique apparente est le rapport entre la masse des microbilles et le volume apparent.

En outre, en jouant sur la taille des microbilles et le débit de passage, des cristaux plus au moins fins peuvent être obtenus. Par exemple, un broyage plus fin pourra être obtenu si le débit de la suspension de départ est ralenti.

L'étape de broyage peut être réalisée en mode continu ou mode discontinu en un ou plusieurs passages (mode pendulaire ou en recirculation).

Lorsqu'elle est réalisée en mode discontinu, le nombre de passages de la suspension dite de départ peut être de 1 à 10, préférentiellement de 1 à 5 (à savoir, après un premier passage, on récupère la suspension à la sortie 6 et on l'a réinjecte de nouveau, grâce à la pompe, dans la chambre 2 via l'entrée 5 pour permettre un deuxième passage). En particulier, le nombre de passages de la suspension de départ est de 1.

En effet, la Demanderesse a remarqué qu'un seul et unique passage dans le broyeur à microbilles, malgré un temps de séjour très court, permettait d'obtenir en sortie 6 une suspension comprenant très majoritairement des cristaux de diglycéroxyde de calcium (notamment lorsque les matières de départ sont en proportion stœchiométrique), de pureté et de taille très satisfaisantes.

Ainsi, cette étape de broyage sera réalisée de préférence en mode continu.

Avantageusement, cette étape de broyage se déroule à une température ambiante inférieure ou égale à 50°C, à savoir le plus souvent à une température ambiante allant de 15°C à 45°C, en particulier de 18°C à 35°C et en général est de l'ordre de 20°C à 25°C. En effet, le procédé selon l'invention ne nécessite pas de chauffage particulier afin de fabriquer les cristaux de diglycéroxyde de calcium et ce, contrairement à l'enseignement divulgué dans les arts antérieurs. Ainsi, les réactifs de départ /suspension de départ sont/est introduit(s) dans le broyeur à température ambiante (généralement aux alentours de 20-25°C). Cependant, il se peut qu'en fonction des réactifs de départ utilisés, une réaction exothermique ait lieu au cours de cette étape de broyage et que la température s'élève légèrement (généralement la température en sortie du broyeur est inférieure ou égale à 50°C).

Une fois l'étape de broyage réalisée, (3) la suspension de diglycéroxyde de calcium est récupérée à la sortie 7 du broyeur 1 et est éventuellement (4) lavée des traces de glycérol en excès à l'aide de méthanol, et optionnellement (5) séchée de sorte à obtenir une poudre de diglycéroxyde de calcium.

Selon une variante de réalisation, la suspension de diglycéroxyde de calcium peut être séchée à l'air libre. Il est en effet apparu que la réaction de carbonatation est très lente entre le diglycéroxyde de calcium et le CO₂ de l'air.

Selon une autre variante de réalisation, la suspension de diglycéroxyde de calcium peut être séchée à une température de 50 à 100°C pendant 1 à 4 heures.

Le produit final est généralement conservé jusqu'à son utilisation sous forme de pâte ou mélangé à un solvant. Il peut aussi être récupéré sous forme solide et conservé dans un récipient étanche.

Comme cela sera démontré dans les essais ci-dessous, on obtient des cristaux de diglycéroxyde de calcium homogènes, globalement de forme parallépipédique.

Les cristaux de diglycéroxyde de calcium obtenus par le procédé de l'invention présente généralement les répartitions de population suivantes en fonction de leur granulométrie mesurées avec un granulomètre laser en voie liquide :
0,5µm ≤ Dₓ50 ≤50µm, de préférence 1 µm ≤ Dₓ50 ≤10µm et
1 µm ≤ Dₓ90 ≤ 75µm, de préférence 5µm ≤ Dₓ90 ≤ 40µm.

En outre, telles que mentionnées ci-dessus, les caractéristiques des cristaux de diglycéroxyde de calcium (taille, épaisseur, etc.) pourront être ajustées, en fonction des nécessités de l'utilisation envisagée, en faisant varier quelques paramètres, tels que le débit de passage, le diamètre des microbilles ou encore la teneur en solvant anhydre du glycérol dans la suspension de départ.

La présente invention peut avoir pour objet l'utilisation des cristaux de diglycéroxyde de calcium obtenus selon le procédé susmentionné.

Le diglycéroxyde de calcium peut être utilisé comme catalyseur hétérogène pour la production de biodiesel comme cela est mentionné dans la publication « Direct synthesis of calcium glyceroxide from hydrated lime and glycerol and its evaluation in the transesterification reaction » publiée dans le revue « Fuel 138 (2014) - 126 à 133 ».

En particulier, le diglycéroxyde de calcium peut être utilisé en tant que catalyseur hétérogène pour la production de biodiesel par réaction du méthanol avec des huiles végétales.

D'autres applications de catalyse sont envisageables, notamment celles nécessitant l'utilisation d'un catalyseur à caractère basique au sens de Bronsted.

### EXEMPLES

La description des essais ci-dessous est donnée à titre d'exemple purement illustratif et non limitatif.

### A° Caractérisation

### ✔ MEB

La microscopie électronique à balayage (MEB) a été effectuée sur un appareil Zeiss EVO MA 15, en électrons secondaires et rétrodiffusés (contraste chimique) avec un faisceau primaire de 5 à 20 kV.

Pour réaliser les essais par MEB, les suspensions de cristaux de diglycéroxyde de calcium ont été préalablement séchées à l'air à 50°C de manière à obtenir une poudre.

### ✔ DRX

Les spectres de diffractométrie de rayons X (DRX) ont été collectés avec un diffractomètre D8 ADVANCE Série II commercialisé par la société Bruker en utilisant la radiation CuKα1 (0,15406 nm) selon la configuration Bragg-Brentano.

Le détecteur utilisé est un détecteur LynxEye 1D de chez Bruker. L'angle d'ouverture du détecteur est de 3° (150 bandes).

Les mesures DRX ont été réalisées entre 10° et 120° (à l'échelle 2θ) avec un pas de 0,008° (1 s /pas).

Pour réaliser les essais par DRX, les suspensions de cristaux de diglycéroxyde de calcium ont également été préalablement séchées à l'air à 50°C, de manière à obtenir une poudre. Quatre scans consécutifs ont été réalisés afin de confirmer l'évolution temporelle de la poudre étudiée.

### ✔ GRANULOMETRIE EN VOIE LIQUIDE

Les mesures de granulométrie en voie liquide ont été réalisées avec un granulomètre laser de la société Malvern Modèle Mastersizer 3000E.

Pour cette mesure, 5cm³ des échantillons de suspension de cristaux de diglycéroxyde de calcium à analyser ont été préalablement dispersés dans un récipient présentant un diamètre de 40 mm à l'aide d'un générateur d'ultra-sons 28khz/40khz muni d'une sonotrode de diamètre de 20mm.

Cette mesure donne la répartition en volume (en %) des particules de l'échantillon pour chaque classe granulométrique en µm (qui correspond à la longueur la plus longue des cristaux de diglycéroxyde de calcium).

### ✔ ATD/ATG

Les analyses thermiques différentielles (ADT) et thermogravimétriques (ATG) ont été réalisées avec un appareil mixte ATD/ATG de la société Linseis, modèle STA PT1000.

Les analyses ont été effectuées sous air, avec une rampe de température de 10°c/min, allant de 20°c à 900°c.

Pour réaliser les essais, les suspensions de cristaux de diglycéroxyde de calcium ont été préalablement séchées à l'air à 50°C, de manière à obtenir une poudre.

### B° Procédure de préparation des échantillons testés

### ✔ Appareillage

Les essais ont été mis en œuvre dans un broyeur tridimensionnel à microbilles Dyno Mill MultiLab de la Société Willy A. Bachofen AG qui contient 1 KG de microbilles.

Les microbilles sont en oxyde de zirconium et présentent un diamètre de 0.5, 1,0 ou 2,0 mm. Les caractéristiques des microbilles utilisées pour les essais sont résumées dans le tableau 2 ci-dessous :

**Tableau 2**

| **Billes** | **500µm** | **1,0mm** | **2,00mm** |
|---|---|---|---|
| Composition (% en masse) | 93% ZrO₂ | 93% ZrO₂ | 93% ZrO₂ |
| | 5% Y₂O₃ | 5% Y₂O₃ | 5% Y₂O₃ |
| | 2% autres | 2% autres | 2% autres |
| Masse volumique spécifique | 6g/cm³ | 6g/cm³ | 6g/cm³ |
| Masse volumique apparente | 3,7 kg/L | 3,7 kg/L | 3,7 kg/L |
| Dureté de Vickers | 1250 HV1 | 1250 HV1 | 1250 HV1 |

Les microbilles de 500 µm sont notamment commercialisées sous le nom de marque Zirmil® Y Ceramic Beads par la société Saint-Gobain.

La chambre de broyage du broyeur présente une capacité de 309 mL et est remplie, en volume, par rapport à son volume total et en fonction des essais, de 80% des microbilles décrites ci-dessus.

En fonctionnement, les microbilles sont mises en agitation par un agitateur à une vitesse de rotation de 2890 tr/min (où 1 tr/min = 1/60 Hz).

L'agitateur comporte en outre deux disques mélangeurs en polyuréthane de 64mm de diamètre.

### ✔ Matières premières

Pour les essais, les matières premières de départ sont : de l'oxyde de calcium (CaO) présentant une pureté ≥ 99% commercialisé par la société Sigma Aldrich, et du glycérol de pureté ≥ 99.5% commercialisé par la société Mondroguiste.com.

### ✔ Procédure générale mise en œuvre pour les essais :

Pour réaliser chaque essai ci-dessous, les étapes suivantes sont réalisées :
- une suspension de départ est préparée dans un Bécher à partir de l'oxyde de calcium et de glycérol, en proportion stœchiométrique, dans un solvant anhydre du glycérol (méthanol), puis la suspension de départ est mise sous agitation à l'aide d'un agitateur magnétique ;
- elle est ensuite amenée, via une pompe péristaltique à débit réglable au broyeur Dyno Mill MultiLab décrit ci-dessus : le débit de passage dans le broyeur peut atteindre 60L/h. Dans cet essai, il a été fixé à 45L/h correspondant à un temps de séjour de 8s ;
- la suspension de départ est ensuite broyée dans le broyeur comportant des microbilles de 0,5 mm ; de 1mm ou de 2mm de diamètre pendant une certaine durée (qui dépend du débit de passage de la suspension de départ) à température ambiante (20-25°C), permettant ainsi, à la sortie du broyeur, l'obtention d'une suspension de cristaux de diglycéroxyde de calcium ;
- enfin, la suspension de cristaux de diglycéroxyde de calcium est récupérée.

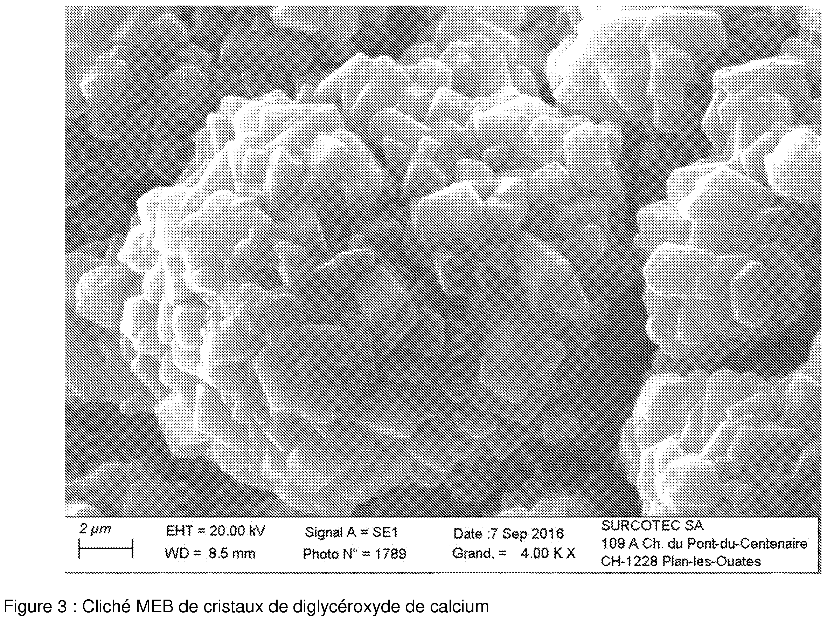

### C° Caractérisation des cristaux de diglycéroxyde de calcium obtenus (figures 3, 4 et 5)

Comme on peut le voir sur la figure 3, selon le procédé de l'invention, après seulement un passage dans le broyeur à microbilles et ainsi un temps de séjour de 8 secondes, les cristaux de diglycéroxyde de calcium sont obtenus (au lieu de temps beaucoup plus longs, pouvant atteindre plusieurs heures, selon les procédés de l'art antérieur).

Les paramètres suivants ont été utilisés : suspension de départ comprenant 980g de matières premières (glycérol + CaO avec un ratio molaire (glycérol) : CaO égal à 5, dans 1kg de solvant anhydre du glycérol (ici, le méthanol) ; un débit de passage de la suspension de départ dans le broyeur de 45L/h et un diamètre de billes de 500 µm ;

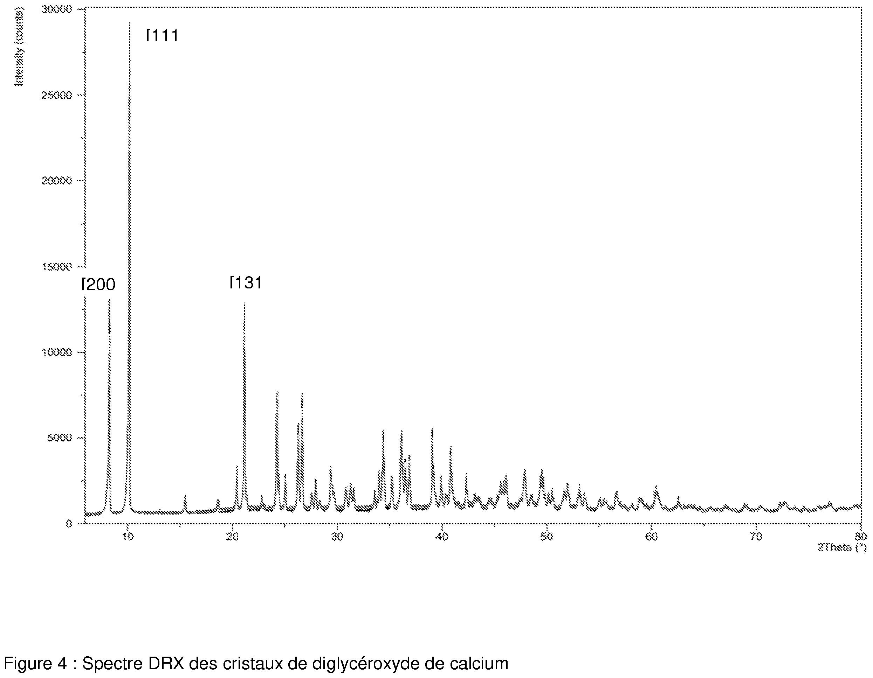

En se référant à la figure 4, on s'aperçoit que les lignes des quatre spectres DRX, scannés successivement, sont tout à fait conformes à celles du spectre de référence du diglycéroxyde de calcium, connu de l'homme du métier (dépôt CDDC réf #828033), aussi bien pour leurs positions angulaires que pour leurs intensités relatives, à savoir :
- On note la présence des trois pics principaux (plans cristallographiques [200], [111] et [131]), à respectivement 8,281°/ 10,177° et 21,179°,
- Tous les autres pics significatifs sont présents aux angles prévus, tels que 20,436°/ 24,466°/ 25,071°/ 26,671°/ 27,973°/ 34,446°/ 36,505°/ 36,894°/ 39,130°/ 40,850° ; avec sensiblement les intensités prévues,

Ces spectres DRX montrent également que l'échantillon analysé présente une excellente pureté. En effet, aucune contamination n'est répertoriée :
- les pics principaux de CaO (selon JCPDS 037-1481), qui sont à 30,204°/ 37,347° et 53,856° n'y apparaissent pas;
- les pics principaux de Ca(OH)2 (selon JCPDS 044-1481), qui sont à 18,008° / 28,672° /34,102° n'y apparaissent pas non plus;
- et le pic principal de CaCO3 (selon JCPDS 005-0586) qui est à 29,4° n'est pas présent de manière significative.

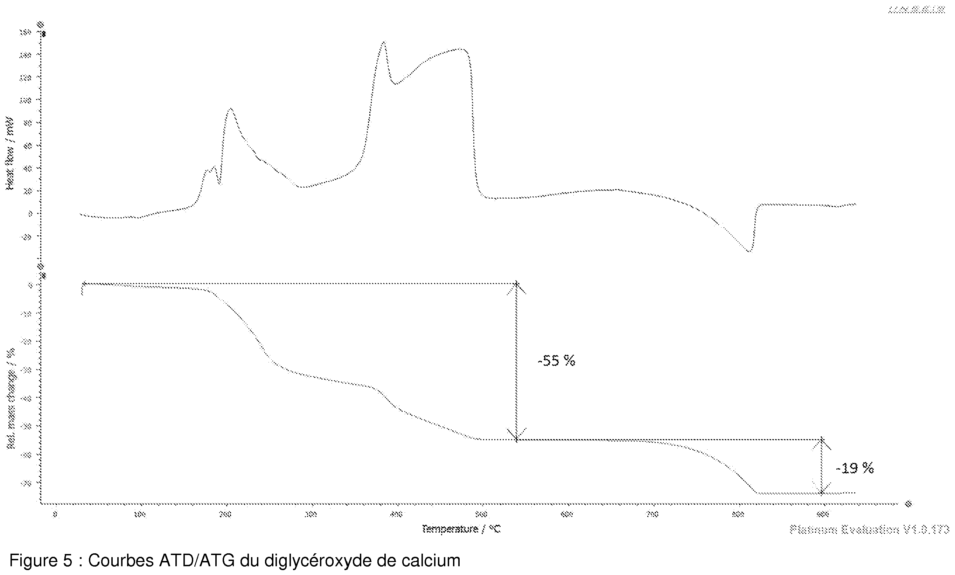

Enfin, les spectres ATG/ATD présentés en figure 5 sont conformes au comportement des cristaux de diglycéroxyde de calcium. Celui-ci a notamment été décrit par L. Leon-Reina et al. dans la publication intitulée « Structural and surface study of calcium glyceroxide, an active phase for biodiesel production under heterogeneous catalysis » datant de 2013 et parue dans le Journal of Catalysis 300. On retrouve ainsi les deux évolutions caractéristiques :
- Une perte de masse d'environ 55% entre 180°c et 500°c, qui peut correspondre à la décomposition du diglycéroxyde de calcium en carbonate de calcium, qui entrainerait une perte de masse théorique de 55%.
- Une perte de masse d'environ 19% entre 700°c et 850°c, qui peut correspondre à la décarbonatation du carbonate de calcium en oxyde de calcium, qui serait marquée par une perte de masse théorique de 19.8%.

En conclusion, le procédé de l'invention permet d'obtenir immédiatement des cristaux de diglycéroxyde de calcium très purs.

### D° Influence du diamètre et du type de billes

Cet exemple a pour but de déterminer si la taille et la composition des billes utilisées lors de la mise en œuvre du procédé ont une influence sur la qualité des cristaux de diglycéroxyde de calcium obtenus.

De la même manière que pour les essais de l'exemple précédent, les cristaux de diglycéroxyde de calcium ont été synthétisés suivant la procédure générale mentionnée ci-dessus et en utilisant en particulier les paramètres suivants :
- Une suspension de départ contenant 980g de matières premières (CaO + glycérol) avec un ratio molaire (glycérol) : (CaO) égal à 5, pour 1kg de solvant anhydre du glycérol (méthanol) ;
- Un débit de passage de 45L/h ;
- Des billes de broyage de 500µm ou 2mm de diamètre, pouvant être majoritairement composées de SiO₂ (Silibeads ® type S) ou majoritairement composées de ZrO₂ (Zirmil® Y Ceramic Beads) ;
- Et n'effectuant qu'un seul passage dans le broyeur ;

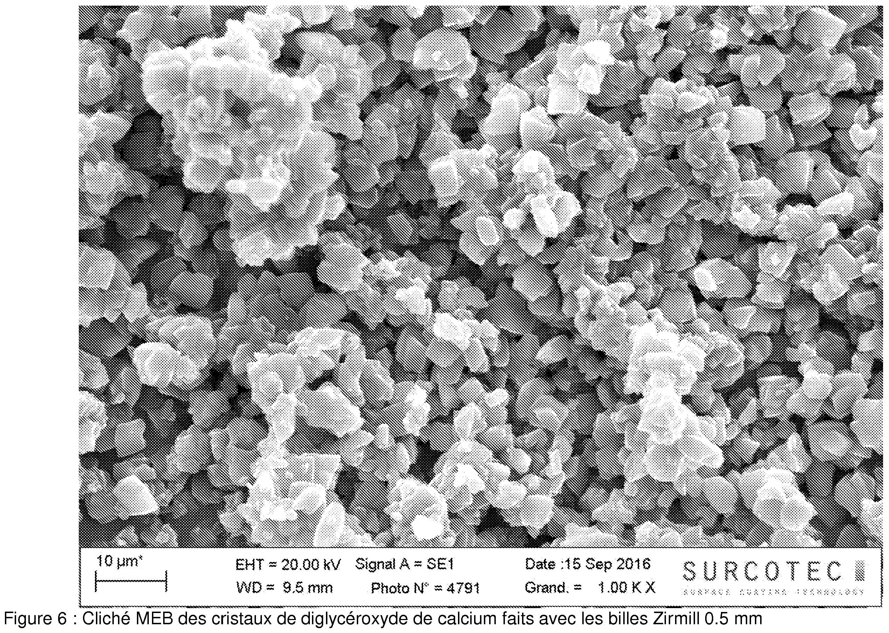

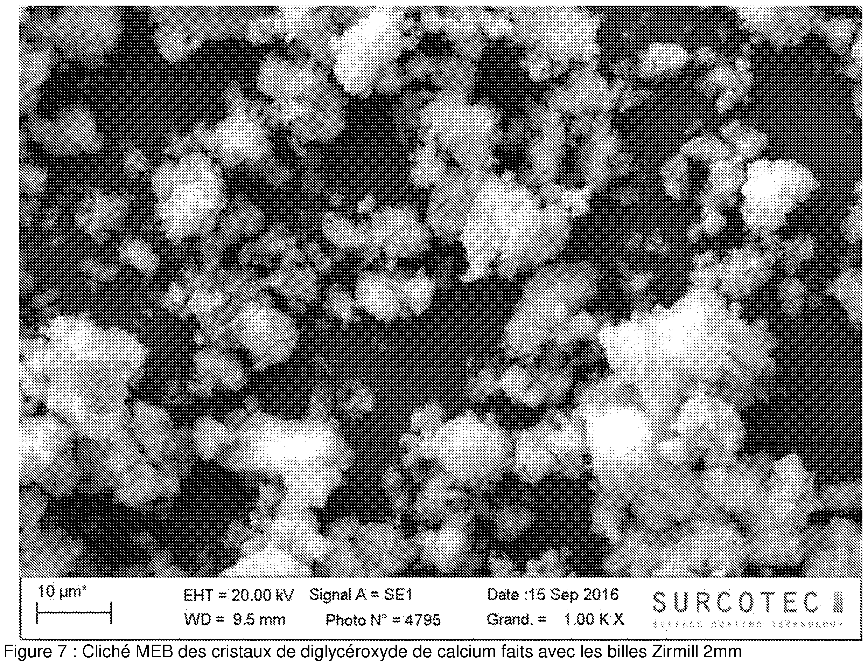

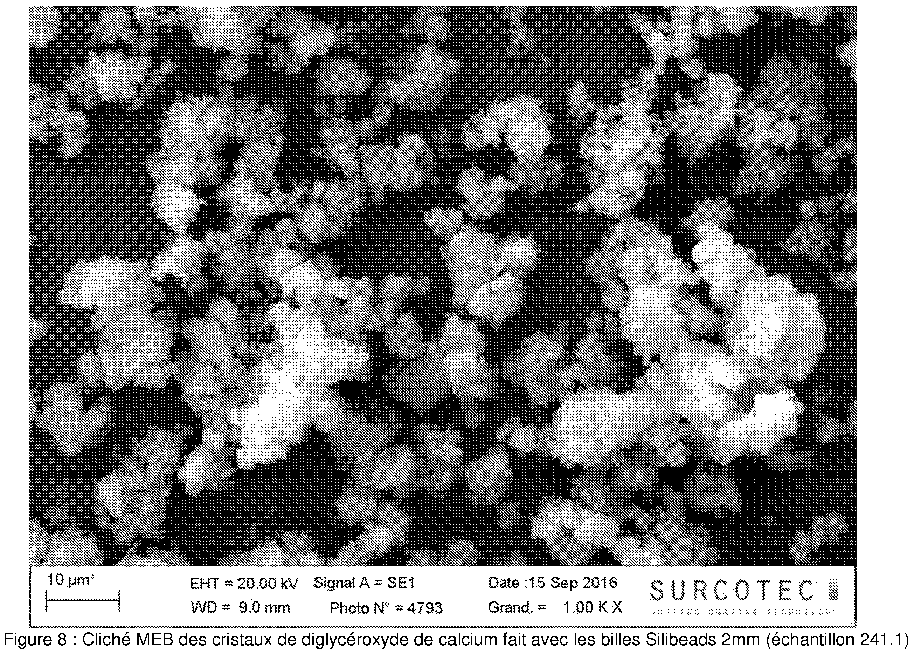

Les résultats de ces essais sont illustrés sur les figures 6 à 8.

D'après les clichés MEB, on constate que la conversion de l'oxyde de calcium et du glycérol en diglycéroxyde de calcium est très proche de 100%, quelle que soit la taille ou la composition des billes utilisées.

Egalement, il a été constaté que quel que soit le type de billes ou leur diamètre, la taille des cristaux de diglycéroxyde de calcium est sensiblement constante. En particulier, on obtient la répartition granulométrique suivante (tableau 3) :

**Tableau 3**

| **Essais** | Dx(10) (µm) | Dx(50) (µm) | Dx(90) (µm) |
|---|---|---|---|
| 1) Billes ZrO₂ - 500µm | 0,96 | 2,4 | 8,69 |
| 2) Billes SiO₂ - 500µm | 1,08 | 2,85 | 9,10 |
| 3) Billes ZrO₂ - 2mm | 1,12 | 3,13 | 8,69 |
| *Moyenne* | *1,05* | *2,79* | *8,83* |
| *Ecart type* | *0,08* | *0,37* | *0,24* |

Ainsi, le procédé, selon l'invention, permet d'obtenir des cristaux de diglycéroxyde de calcium homogènes de petites tailles (Dx(50) de préférence entre 1 et 10 µm et particulièrement entre 1 et 5µm).

### E/ Granulométrie des cristaux de diglycéroxyde de calcium en fonction du nombre de passages.

Pour cet exemple, différents nombres de passages dans le broyeur tridimensionnel ont été testés (1 et 45 passages), afin de vérifier leur impact sur la granulométrie des cristaux de diglycéroxyde de calcium obtenus.

Ainsi, les cristaux de diglycéroxyde de calcium testés ont été synthétisés suivant la procédure générale mentionnée ci-dessus et en utilisant les paramètres suivants :
- Une suspension de départ contenant 306g de matières premières (CaO + glycérol) avec un ratio molaire (glycérol) : (CaO) égal à 2, pour 1kg de solvant anhydre du glycérol (méthanol) ;
- Un débit de passage de 45L/h ;
- Des billes de broyage de 500µm majoritairement composées de ZrO₂ (Zirmil® Y Ceramic Beads) ;
- Et effectuant un seul ou 45 passages dans le broyeur ;

La répartition granulométrique est la suivante (Tableau 4) :

**Tableau 4**

| **Essais** | Dx(10) (µm) | Dx(50) (µm) | Dx(90) (µm) |
|---|---|---|---|
| 1 passage | 1,08 | 3,23 | 9,99 |
| 45 passages | 0,41 | 0,82 | 6,43 |

On constate qu'un plus grand nombre de passage permet d'obtenir des cristaux de plus petite taille.

Ainsi, le procédé selon l'invention permet d'obtenir des cristaux de diglycéroxyde de calcium, dont la taille pourra être adaptée facilement en modifiant le nombre de passages dans le broyeur.

### F° Caractérisation des cristaux de diglycéroxyde de calcium obtenus en utilisant une autre source de calcium

Pour cet exemple, une autre source minérale contenant l'élément calcium, à savoir Ca(OH)₂, a été utilisée pour synthétiser les cristaux de diglycéroxyde de calcium.

La procédure générale mentionnée ci-dessus a été appliquée en utilisant les paramètres suivants :
- Une suspension de départ contenant 1072g de matières premières (Ca(OH)₂ + glycérol) avec un ratio molaire (glycérol) : (Ca(OH)₂) égal à 5, pour 1kg de solvant anhydre du glycérol (méthanol) ;
- Un débit de passage de 45L/h ;
- Des billes de broyage de 500µm majoritairement composées de ZrO₂ (Zirmil® Y Ceramic Beads) ;
- Et effectuant un seul passage dans le broyeur.

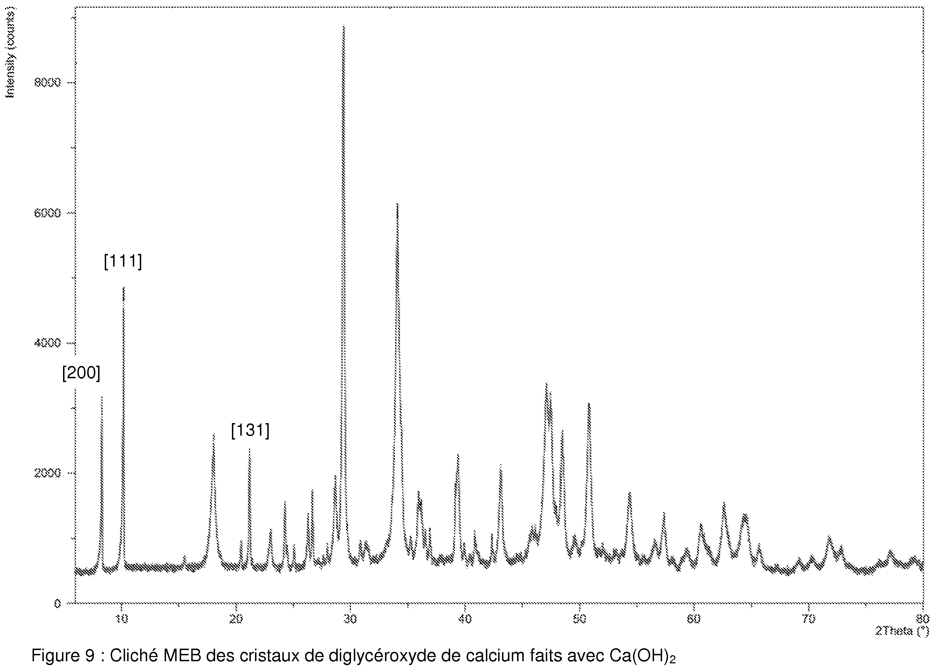

En se référant à la figure 9, on s'aperçoit que les lignes des quatre spectres DRX, scannés successivement, sont tout à fait conformes à celles du spectre de référence du diglycéroxyde de calcium, connu de l'homme du métier (dépôt CDDC réf #828033), aussi bien pour leurs positions angulaires que pour leurs intensités relatives, à savoir :
- On note la présence des trois pics principaux (plans cristallographiques [200], [111] et [131]), à respectivement 8,281°/ 10,177° et 21,179°,
- Tous les autres pics significatifs sont présents aux angles prévus, tels que 20,436°/ 24,466°/ 25,071°/ 26,671°/ 27,973°/ 34,446°/ 36,505°/ 36,894°/ 39,130°/ 40,850° ; avec sensiblement les intensités prévues,

Ces spectres DRX montrent également la présence résiduelle de matière non transformée. En effet :
- les pics principaux de Ca(OH)2 (selon JCPDS 044-1481), qui sont à 18,008° / 28,672° /34,102° sont présents;
- et le pic principal de CaCO3 (selon JCPDS 005-0586) qui est à 29,4° est également observé

Ainsi, il a été montré que les cristaux de diglycéroxyde de calcium peuvent être synthétisés en utilisant Ca(OH)₂ en tant que matière première source de l'élément calcium. Particulièrement, dans les conditions de la procédure générale mentionnée, à savoir avec un seul passage dans le broyeur pour un débit de 45L/h, il a été constaté que les cristaux de diglycéroxyde de calcium obtenus avec Ca(OH)₂ sont légèrement moins purs que ceux obtenus dans des conditions similaires avec CaO (exemple C). Cependant, une conversion approchant 100% pour Ca(OH)₂ est envisageable, notamment en augmentant le temps de séjour dans le broyeur.

### G° Conclusion

Ainsi, il a été démontré que pour tous les essais, une poudre insoluble dans le solvant anhydre du glycérol est obtenue. Après contrôle par analyse DRX, il est apparu que cette poudre correspondait à des cristaux de diglycéroxyde de calcium (tous les spectres DRX effectués par la Demanderesse montrent une transformation des composés de départ en diglycéroxyde de calcium, les spectres indiquent en effet les raies correspondant au diglycéroxyde de calcium, avec seulement quelques traces résiduelles des composés initiaux).

Les courbes ATD/ATG réalisées se sont révélées en totale adéquation avec les éléments de l'état de l'art bien connus de l'Homme du métier, avec notamment une perte de masse totale après 850°c de 75%, pouvant correspondre à la décomposition du diglycéroxyde de calcium en oxyde de calcium (74.8%).

Les clichés obtenus par microscopie à balayage électronique mettent en évidence des cristaux diglycéroxyde de calcium. Il s'avère également que la poudre obtenue est principalement constituée de cristaux bien formés dont la répartition granulométrique Dx(50) est généralement inférieure à 10µm.

Bien évidemment en fonction des paramètres de broyage, par exemple en jouant sur les dimensions des microbilles, on peut obtenir des cristaux de dimensions plus importante, (Dx50) jusqu'à 50µm) ou moins importante (Dₓ(50) <1µm).

En outre, le broyeur de laboratoire permet par exemple de produire 54kg/h de cristaux de diglycéroxyde en calcium. Ce chiffre pourrait être multiplié par 10 avec l'adjonction d'un accessoire accélérateur. Egalement, il existe des versions industrielles du broyeur utilisant par exemple jusqu'à 100 kg de billes. Avec ce type de broyeur, il serait par conséquent possible de fabriquer plusieurs tonnes à l'heure de diglycéroxyde de calcium.

## Revendications

1. Procédé de fabrication de cristaux de diglycéroxyde de calcium qui comprend au moins les étapes suivantes :
(1) la mise en suspension d'au moins un composé source de l'élément calcium, notamment l'oxyde de calcium (CaO), dans du glycérol ou dans un mélange homogène de glycérol (C₃H₈O₃) et d'un solvant anhydre du glycérol, notamment du méthanol (CH₃OH), dite « suspension de départ », le ratio molaire (glycérol) : (composé(s) source de l'élément calcium) étant supérieur ou égal à 2, de préférence de 2 à 10, et en particulier de 2 à 5 ;
(2) le broyage de ladite suspension de départ à une température ambiante inférieure ou égale à 50°C, de préférence inférieure ou égale à 35°C, dans un broyeur tridimensionnel à microbilles en phase liquide pendant un temps de séjour inférieur ou égal à 15 min, de préférence inférieur ou égal à 1 minute, et allant notamment de 5 à 25 secondes et en particulier de 10 à 20 secondes ;
(3) la récupération en sortie du broyeur d'une suspension de cristaux de diglycéroxyde de calcium, et
(4) optionnellement, un lavage de la suspension obtenue avec un solvant du glycérol afin d'éliminer le possible excès de glycérol,
(5) optionnellement, ladite suspension de cristaux de diglycéroxyde de calcium est séchée de sorte à obtenir une poudre de cristaux de diglycéroxyde de calcium.

2. Procédé selon la revendication 1, dans lequel le ratio molaire (solvant anhydre du glycérol) : (glycérol) va de 0 à 30, plus particulièrement de 2 à 15 et de préférence de 3 à 12.

3. Procédé selon les revendications 1 ou 2, dans lequel le glycérol et le(les) composé(s) source de l'élément calcium sont mélangés en proportion stœchiométrique, en présence ou non de solvant anhydre du glycérol.

4. Procédé selon l'une quelconque des revendications 1 à 3, dans lequel le composé source de l'élément calcium est l'oxyde de calcium CaO, Ca(OH)₂, l'acétate de calcium ou encore le méthanolate de calcium, ou une de leurs combinaisons.

5. Procédé selon l'une quelconque des revendications 1 à 4, dans lequel le solvant anhydre du glycérol est le méthanol (CH₃OH), l'isopropanol et l'éthanol et est de préférence le méthanol.

6. Procédé selon l'une des revendications précédentes, dans lequel les microbilles sont de forme sphérique et présentent un diamètre moyen allant de 0,05 mm à 4 mm, de préférence de 0,2 à 3 mm, en particulier de 0,3 à 2 mm et typiquement de l'ordre de 0,5 à 1 mm.

7. Procédé selon l'une des revendications précédentes, dans lequel les microbilles présentent une dureté de Vickers mesurée selon la norme EN ISO 6507-1 supérieure ou égale à 900 HV1, de préférence allant de 900 HV1 à 1600 HV1, typiquement allant de 1000 à 1400 HV1.

8. Procédé selon l'une des revendications précédentes, dans lequel les microbilles présentent une masse volumique réelle allant de 2 à 15 g/cm³.

9. Procédé selon l'une des revendications précédentes, dans lequel le broyage s'effectue à une température ambiante allant de 15°C à 45°C, en particulier de 18°C à 35°C et en général est de l'ordre de 20°C à 25°C.

10. Procédé selon l'une des revendications précédentes, dans lequel le broyeur tridimensionnel à microbilles comprend au moins :
- une chambre de broyage stationnaire de forme générale cylindrique s'étendant selon un axe longitudinal XX, ladite chambre étant remplie au moins, en partie, par lesdites microbilles et comprend: à une première extrémité au moins une entrée servant à introduire ladite suspension de départ, et à une seconde extrémité, une sortie comportant un moyen de séparation apte à n'évacuer que la suspension de diglycéroxyde de calcium formée dans ladite chambre ; et
- un agitateur, disposé dans la chambre de broyage stationnaire, se présentant sous forme d'une tige allongée selon l'axe longitudinal XX, ledit agitateur étant apte à mettre en mouvement l'ensemble microbilles/suspension de départ.

11. Procédé selon la revendication 10, dans lequel les microbilles représentent, en volume, par rapport au volume total de la chambre stationnaire de 50% à 85%, de préférence de 55% à 70%.

12. Procédé selon l'une des revendications précédentes, dans lequel le broyeur fonctionne en continu.

13. Procédé selon l'une des revendications 10 à 12, dans lequel la vitesse de rotation de l'agitateur est supérieure ou égale à 100 tours par minute.

14. Procédé selon l'une des revendications précédentes, dans lequel la suspension de départ présente un temps de séjour dans le broyeur tridimensionnel à microbilles en phase liquide inférieur ou égal à 1 minute.

15. Procédé selon l'une des revendications précédentes, dans lequel les microbilles sont choisies parmi : des microbilles en céramique, des microbilles en acier, des microbilles en carbure de tungstène, des microbilles en verre ou une de leurs combinaisons.

## Patentansprüche

1. Verfahren zur Herstellung von Calciumdiglyceroxidkristallen, das zumindest folgende Schritte umfasst:
(1) Suspendieren von mindestens einer Ausgangsverbindung des Elements Calcium, insbesondere Calciumoxid (CaO), in Glycerol oder in einer homogenen Mischung aus Glycerol (C₃H₈O₃) und einem wasserfreien Glycerollösungsmittel, insbesondere Methanol (CH₃OH), bezeichnet als "Ausgangssuspension", wobei das Molverhältnis (Glycerol) : (Ausgangsverbindung(en) des Elements Calcium) größer gleich 2, vorzugsweise 2 bis 10 und insbesondere 2 bis 5 beträgt;
(2) Mahlen der Ausgangssuspension bei einer Umgebungstemperatur von kleiner gleich 50°C, vorzugsweise kleiner gleich 35°C, in einer 3D-Kugelmühle mit Mikrokügelchen in der flüssigen Phase während einer Verweildauer von kürzer als oder gleich 15 min, vorzugsweise kürzer als oder gleich 1 Minute, und die insbesondere von 5 bis 25 Sekunden und vor allem von 10 bis 20 Sekunden dauert;
(3) am Austrag der Mühle Gewinnen einer Suspension mit Calciumdiglyceroxidkristallen, und
(4) gegebenenfalls Waschen der erhaltenen Suspension mit einem Glycerollösungsmittel zum Entfernen des möglichen Glycerolüberschusses,
(5) gegebenenfalls wird die Suspension von Calciumdiglyceroxidkristallen derart getrocknet, dass ein Calciumdiglyceroxidkristall-Pulver erhalten wird.

2. Verfahren nach Anspruch 1, wobei das Molverhältnis (wasserfreies Glycerollösungsmittel) : (Glycerol) von 0 bis 30, insbesondere von 2 bis 15 und vorzugsweise von 3 bis 12 beträgt.

3. Verfahren nach Anspruch 1 oder 2, wobei Glycerol und die Ausgangsverbindung(en) des Elements Calcium in Gegenwart von dem oder ohne das wasserfreie Glycerollösungsmittel im stöchiometrischen Verhältnis gemischt werden.

4. Verfahren nach einem der Ansprüche 1 bis 3, wobei die Ausgangsverbindung des Elements Calcium Calciumoxid CaO, Ca(OH)₂, Calciumacetat oder auch Calciummethanolat oder eine ihrer Kombinationen ist.

5. Verfahren nach einem der Ansprüche 1 bis 4, wobei das wasserfreie Glycerollösungsmittel Methanol (CH₃OH), Isopropanol und Ethanol ist und vorzugsweise Methanol ist.

6. Verfahren nach einem der vorhergehenden Ansprüche, wobei die Mikrokügelchen rund sind und einen mittleren Durchmesser von 0,05 mm bis 4 mm, vorzugsweise von 0,2 bis 3 mm, insbesondere von 0,3 bis 2 mm und typischerweise in der Größenordnung von 0,5 bis 1 mm aufweisen.

7. Verfahren nach einem der vorhergehenden Ansprüche, wobei die Mikrokügelchen eine nach der Norm EN ISO 6507-1 gemessene Vickers-Härte von größer gleich 900 HV1 aufweisen, die vorzugsweise von 900 HV1 bis 1600 HV1, typischerweise von 1000 bis 1400 HV1 beträgt.

8. Verfahren nach einem der vorhergehenden Ansprüche, wobei die Mikrokügelchen eine wahre Dichte zwischen 2 und 15 g/cm³ aufweisen.

9. Verfahren nach einem der vorhergehenden Ansprüche, wobei das Mahlen bei einer Umgebungstemperatur zwischen 15°C und 45°C, insbesondere zwischen 18°C und 35°C erfolgt und im Allgemeinen in der Größenordnung von 20°C bis 25°C liegt.

10. Verfahren nach einem der vorhergehenden Ansprüche, wobei die 3D-Mühle mit Mikrokügelchen zumindest umfasst:
- eine stationäre Mahlkammer mit im Allgemeinen zylindrischer Form, die in einer Längsachse XX verläuft, wobei die Kammer zumindest teilweise mit den Mikrokügelchen gefüllt ist und umfasst: an einem ersten Ende mindestens einen Einlass, der zum Einleiten der Ausgangssuspension dient, und an einem zweiten Ende einen Auslass, der ein Trennmittel aufweist, mit dem sich ausschließlich die in der Kammer entstandene Calciumdiglyceroxidsuspension ableiten lässt, und
- einen in der stationären Mahlkammer angeordneten Rührer, der in Form eines länglichen Stabs in Richtung der Längsachse XX vorliegt, wobei der Rührer in der Lage ist, die Gesamtheit aus Mikrokügelchen/Ausgangssuspension in Bewegung zu versetzen.

11. Verfahren nach Anspruch 10, wobei die Mikrokügelchen bezogen auf das Gesamtvolumen der stationären Kammer 50 Volumen-% bis 85 Volumen-%, vorzugsweise 55 Volumen-% bis 70 Volumen-% ausmachen.

12. Verfahren nach einem der vorhergehenden Ansprüche, wobei die Mühle kontinuierlich arbeitet.

13. Verfahren nach einem der Ansprüche 10 bis 12, wobei die Drehzahl des Rührers größer gleich 100 Umdrehungen pro Minute beträgt.

14. Verfahren nach einem der vorhergehenden Ansprüche, wobei die Ausgangssuspension eine Verweildauer in der 3D-Mühle mit Mikrokügelchen in der flüssigen Phase von kürzer als oder gleich 1 Minute aufweist.

15. Verfahren nach einem der vorhergehenden Ansprüche, wobei die Mikrokügelchen ausgewählt werden aus: Keramik-Mikrokügelchen, Stahl-Mikrokügelchen, Wolframcarbid-Mikrokügelchen, Glas-Mikrokügelchen oder einer ihrer Kombinationen.

## Claims

1. Method for manufacturing calcium diglyceroxide crystals that comprises at least the following steps:
(1) placing at least one calcium element source compound, especially calcium oxide (CaO), in suspension in glycerol or in a homogeneous mixture of glycerol (C₃H₈O₃) and an anhydrous glycerol solvent, especially methanol (CH₃OH), referred to as the "starting suspension", the molar ratio of (glycerol):(calcium element source compound(s)) being greater than or equal to 2, preferably is ranging from 2 to 10, and in particular is ranging from 2 to 5;
(2) milling said starting suspension at an ambient temperature of less than or equal to 50°C, preferably less than or equal to 35°C, in a three-dimensional liquid-phase microbead mill for a holding time of less than or equal to 15 min, preferably less than or equal to 1 minute, and ranging particularly from 5 to 25 seconds and in particular from 10 to 20 seconds;
(3) recovering, at the outlet of the mill, a suspension of calcium diglyceroxide crystals, and
(4) optionally, washing the obtained suspension with a glycerol solvent in order to eliminate any excess glycerol,
(5) optionally, said suspension of calcium diglyceroxide crystals is dried so as to obtain a powder of calcium diglyceroxide crystals.

2. Method according to claim 1, wherein the molar ratio of (anhydrous glycerol solvent):(glycerol) ranges from 0 to 30, more particularly from 2 to 15 and preferably from 3 to 12.

3. Method according to claims 1 or 2, wherein glycerol and the calcium element source compound(s) are mixed in stoichiometric proportions, optionally in the presence of anhydrous glycerol solvent.

4. Method according to any one of claims 1 to 3, wherein the calcium element source compound is calcium oxide CaO, Ca(OH)₂, calcium acetate or calcium methanolate, or any of the combinations thereof.

5. Method according to any one of claims 1 to 4, wherein the anhydrous glycerol solvent is methanol (CH₃OH), isopropanol and ethanol and is preferably methanol.

6. Method according to one of the preceding claims, wherein the microbeads are of spherical shape and have a mean diameter ranging from 0.05 mm to 4 mm, preferably from 0.2 to 3 mm, in particular from 0.3 to 2 mm and typically of the order of 0.5 to 1 mm.

7. Method according to one of the preceding claims, wherein the microbeads have a Vickers hardness measured as per the EN ISO 6507-1 standard greater than or equal to 900 HV1, preferably ranging from 900 HV1 to 1600 HV1, typically ranging from 1000 to 1400 HV1.

8. Method according to one of the preceding claims, wherein the microbeads have a true density ranging from 2 to 15 g/cm³.

9. Method according to one of the preceding claims, wherein the milling is carried out at an ambient temperature ranging from 15°C to 45°C, in particular from 18°C to 35°C and in general is of the order of 20°C to 25°C.

10. Method according to one of the preceding claims, wherein the three-dimensional microbead mill comprises at least:
- a stationary milling chamber of general cylindrical shape extending along a longitudinal axis XX, said chamber being filled at least, in part, with said microbeads and comprises: at a first end at least one inlet serving to introduce said starting suspension, and at a second end, an outlet including a separating means suitable for only discharging the calcium diglyceroxide suspension formed in said chamber; and
- an agitator, arranged in the stationary milling chamber, presented in the form of an elongated rod along the longitudinal axis XX, said agitator being suitable for setting in motion the microbead/starting suspension assembly.

11. Method according to claim 10, wherein the microbeads represent, in volume, with respect to the total volume of the stationary chamber from 50% to 85%, preferably from 55% to 70%.

12. Method according to one of the preceding claims, wherein the mill operates in continuous mode.

13. Method according to one of claims 10 to 12, wherein the rotational speed of the agitator is greater than or equal to 100 revolutions per minute.

14. Method according to one of the preceding claims, wherein the starting suspension has a holding time in the three-dimensional liquid-phase microbead mill less than or equal to 1 minute.

15. Method according to one of the preceding claims, wherein the microbeads are chosen among: ceramic microbeads, steel microbeads, tungsten carbide microbeads, glass microbeads or any of the combinations thereof.
